(19) Europäisches Patentamt
European Patent Office
Office européen des brevets

(11) EP 3 434 365 A1

(12) # EUROPEAN PATENT APPLICATION
published in accordance with Art. 153(4) EPC

(43) Date of publication:
**30.01.2019 Bulletin 2019/05**

(21) Application number: **17769726.5**

(22) Date of filing: **15.02.2017**

(51) Int Cl.:
***B01J 23/89*** *(2006.01)* ***C07C 209/36*** *(2006.01)*
***C07C 209/38*** *(2006.01)*

(86) International application number:
**PCT/JP2017/005582**

(87) International publication number:
**WO 2017/163679 (28.09.2017 Gazette 2017/39)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **23.03.2016 JP 2016058059**

(71) Applicant: **N.E. Chemcat Corporation**
**Tokyo 105-6124 (JP)**

(72) Inventors:
- **MIZUSAKI Tomoteru**
  **Bando-shi**
  **Ibaraki 306-0608 (JP)**
- **SUZUKA Hiroyasu**
  **Bando-shi**
  **Ibaraki 306-0608 (JP)**
- **NAKAYA Yusuke**
  **Bando-shi**
  **Ibaraki 306-0608 (JP)**
- **WADA Yoshiyuki**
  **Bando-shi**
  **Ibaraki 306-0608 (JP)**
- **TAKAGI Yukio**
  **Numazu-shi**
  **Shizuoka 410-0314 (JP)**

(74) Representative: **Mewburn Ellis LLP**
**City Tower**
**40 Basinghall Street**
**London EC2V 5DE (GB)**

(54) **CATALYST MIXTURE**

(57) Provided is a catalyst mixture which, in a nitro group hydrogenation reaction of an aromatic nitro compound having a structure in which nitro groups and halogen atoms are directly bonded as substituents to a ring skeleton of the same ring while separated from each other, is capable of selectively hydrogenating the nitro groups and sufficiently reducing the removal of the halogen atoms from the ring. This catalyst mixture includes a catalyst which is used in a hydrogenation reaction of at least one among one or more nitro groups present in a reactant, which is an aromatic nitro compound having a structure in which one or more nitro groups and one or more halogen atoms are directly bonded as substituents to a ring skeleton of the same ring while separated from each other. This catalyst mixture further includes a base. The catalyst includes a support, and Pt particles and Fe oxide particles supported on the support. The base has a stronger basicity than at least one aromatic amine obtained as a product from the hydrogenation reaction and having at least one amino group.

EP 3 434 365 A1

## Description

### TECHNICAL FIELD

The present invention relates to a catalyst mixture which includes a catalyst used in a hydrogenation reaction of one or more nitro groups of an unsaturated cyclic compound (aromatic nitro compound) having a structure where one or more nitro groups are directly bonded the ring skeleton.

### BACKGROUND ARTS

An aromatic halogen amine having a structure where one or more nitro groups and one or more amino groups are directly bonded as substituents to a ring skeleton of the same ring while separated from each other, is an important raw material of a medicine, a dye, an insecticide, a herbicide.

The aromatic halogen amine can be prepared, for example, by the hydrogenation reaction (catalytic hydrogenation reaction) of an aromatic halogen nitro compound having the corresponding chemical structure (aromatic nitro compound having a structure where one or more nitro groups and one or more halogen atoms are directly bonded as substituents to a ring skeleton of the same ring while separated from each other).

In this hydrogenation reaction, in addition to the reduction reaction (desired hydrogenation reaction) where the nitro group is reduced to an amino group, a reaction where a halogen atom is removed from the ring and replaced by a hydrogen atom (undesired side reaction. Hereinafter referred to as "dehalogenation reaction" as necessary) also happened. Hydrogen halide produced by the dehalogenation reaction corrodes the reaction vessel.

In general, it is known that the dehalogenation reaction of an aromatic halogen compound is accelerated under the condition that a noble metal catalyst such as Pt or Pd and a base (NaOH, KOH, $NH_4OH$) coexist (for example, Non-Patent Documents 1 to 6). The reason why accelerating the dehalogenation reaction is that the base neutralizes the hydrogen halide generated by the dehalogenation reaction. This is described, for example, in Non-Patent Document 1, page 25, left column: Formulae (11) to (13) showing Liquid Phase Dechlorination Reaction, Non-Patent Document 3, page 98, left column: Formulae (1) to (3) showing Liquid Phase Dechlorination Reaction.

As explained above, in the hydrogenation reaction of the aromatic halogen nitro compound, it is important to sufficiently reduce the dehalogenation reaction and to selectively reduce the nitro group.

Various proposals have been made for selectively reducing the nitro group of the aromatic halogen nitro compound.

For example, in order to avoid the dehalogenation reaction, Patent Document 1 proposes that the hydrogenation reaction of the aromatic halogen nitro compound (an aromatic nitro derivative having a halogen atom bonded to an aromatic nucleus in Patent Document 1) is carried out in the presence of tungsten carbide and a strong acid (sulfuric acid, phosphoric acid, hydrochloric acid, hydrobromic acid).

Further, in order to selectively proceed the hydrogenation of a nitro group of the aromatic halogen nitro compound, Patent Document 2 proposes a noble metal catalyst in which Pt and Cu are finely dispersed on an activated carbon as a carrier in a predetermined supporting amount.

Furthermore, in order to selectively proceed the hydrogenation of a nitro group of the aromatic halogen nitro compound (for example, 1-nitro-3,4-dichlorobenzene, 4-nitrochlorobenzene), Patent Document 3 proposes a catalyst containing a Pt-supporting carbon and Fe oxide-supporting carbon (or Fe hydroxide supporting carbon).

Furthermore, in the hydrogenation reaction of a nitro group of the aromatic halogen nitro compound (4-chloro-2,5-dimethoxy-1-nitrobenzene), in order to isolate the reaction product 4-chloro-2,5- dimethoxyaniline without crystallization from the solvent (xylene), Patent Document 4 proposes a reaction system which includes a sulfurized Pt-supporting carbon catalyst, an aliphatic open chain amine (particularly morpholine), an alkaline aqueous solution {compound which gives a pH of 8 to 10 in an aqueous solution (for example, disodium borate, sodium formate, sodium acetate, sodium carbonate, disodium hydrogen phosphate, sodium hydroxide)}, a predetermined amount of water (30 mL in Example 1 of Patent Document 4 , 1.5 mL of water contained in the catalyst), and a predetermined amount of an aromatic organic solvent (675 mL in Example 1 of Patent Document 4)

Incidentally, the present applicant submits, as publications where the above-mentioned publicly-known inventions are described, the following publications:

### PRIOR ART LITERATURE

Patent Document

Patent Document 1: Japanese Patent Laid-Open Application H5-271105
Patent Document 2: Japanese Patent Laid-Open Application H6-71178
Patent Document 3: United State Patent No.4212824
Patent Document 4: Japanese Patent Laid-Open Application H2-45452

Non-Patent Document

Non-Patent Document 1: General Remarks Detoxification of Aromatic chloride compound (Soda and Chloride, 2005, Jan.-Feb.)
Non-Patent Document 2: Y. Ukisu et al., Appl. Catal. A: General., 271(2004) 165-170
Non-Patent Document 3: Y. Ukisu et al., Appl. Catal. B: Environ., 27, 97(2000) 97-104
Non-Patent Document 4: R. H. Mizzoni et al., J. Am, Chem. Soc., 73, 1873(1951)
Non-Patent Document 5: M. M. Robimson, J. Am, Chem. Soc., 80, 6254(1958)
Non-Patent Document 6: E. V. Brown, J. Org. Chem., 30, 1607(1965)

SUMMARY OF INVENTION

Problem to be Solved by the Invention

However, in the hydrogenation reaction of the aromatic halogen nitro compound, from the viewpoint of sufficiently reducing the dehalogenation reaction and selectively reducing the nitro group, the present inventors have found that there is still room for improvement even in the abovementioned conventional technique.
The present invention has been completed considering the abovementioned technical circumstances, and can provide a catalyst mixture which, in a nitro group hydrogenation reaction of an aromatic nitro compound having a structure in which nitro groups and halogen atoms are directly bonded as substituents to a ring skeleton of the same ring while separated from each other, is capable of selectively hydrogenating the nitro groups and sufficiently reducing the removal of the halogen atoms from the ring.

Means for solving the problems

As the results of the present inventors' intensive study for solving the above problems, the present inventors have found that a structure of a catalyst mixture where a catalyst in which, in addition to Pt particles, Fe oxide particles are supported on a carrier is mixed with a predetermined base is effective, and then the present invention has been completed.
More specifically, the present invention is configured by the following technical elements.
Namely, the present invention provides:
(N1) a catalyst mixture comprising a catalyst which is used in a hydrogenation reaction of at least one among one or more nitro groups present in a reactant, which is an aromatic nitro compound having a structure in which one or more nitro groups and one or more halogen atoms are directly bonded as substituents to a ring skeleton of the same ring while separated from each other, which comprises:

a catalyst and a base, and
the catalyst comprises a support, and Pt particles and Fe oxide particles supported on the support, and
the base has a basicity stronger than at least one aromatic amine obtained as a product from the hydrogenation reaction and having one or more amino groups.

In carrying out the selective hydrogenation reaction of the nitro group of the aromatic halogen nitro compound, despite the general recognition of those skilled in the art that addition of a base in this chemical system promotes the dehalogenation reaction as described in the aforementioned Non-Patent Documents 1 to 6, the present inventors have intentionally tried to add a base in a chemical system of a catalyst in which Pt particles and Fe oxide particles coexist, which have not been attempted in the past. As a result, though the detailed mechanism has not been found, in the hydrogenation reaction of the nitro group of the aromatic halogen nitro compound, the catalyst mixture satisfying the above configuration can selectively hydrogenate the nitro group, and the removal of the halogen atom from the ring can be sufficiently reduced.
In the present invention, the "basic" when comparing the basicity between a base and a product (at least one of aromatic amines having one or more amino groups) means "pKa (= - log Ka) ". Here, "Ka" represents a concentration

acid dissociation constant. In the present invention, the "state in which the base has a basicity stronger than the product" means that "the pKa of the base is larger than the pKa of the product". Further, when comparing the pKa of the base with the product pKa, it is not necessary to use the base pKa and the product pKa as measured in the actual reaction system. For comparison of the pKa, the Ka and pKa of each compound defined and described in "Revised 5th Edition Chemical Handbook Basic II (edited by The Chemical Society of Japan)", or the Ka and pKa of each compound described in the stability constant database summarized by the V6 Committee of IUPAC (L. D. Pettit, K. J. Powell, "Stability Constants Database", Academic Software (1997)).

From the viewpoint of obtaining the effect of the present invention more reliably, in the catalyst mixture of the present invention described in (N1), it is preferable that

(N2) the base and a component of a solvent used in the hydrogenation reaction satisfy the condition of the following equation (1):

$$0.90 \leq \{1000 \text{ x } (B/Vs)\} \leq 190.00 \qquad (1)$$

in the equation (1), B being a substance amount (mol) of the base, and Vs being a volume (L) of the organic solvent.

When adjusting an amount of the base contained in the catalyst mixture within the range which satisfies the condition of the above formula (1) with respect to a volume of the organic solvent in the reaction system where the catalyst mixture is used, the effect of the present invention can be more reliably obtained.

Further, from the viewpoint of obtaining the effect of the present invention more reliably, in the catalyst mixture of the present invention described in (N1) or (N2), it is preferable that

(N3) the base and the reactant used in the hydrogenation reaction satisfy the condition of the following equation (2):

$$0.35\% \leq \{100 \text{ x } (B/R)\} \leq 75.50\% \qquad (2)$$

in the equation (2), B being a substance amount (mol) of the base, and R being a substance amount (mol) of the reactant.

When adjusting an amount of the base contained in the catalyst mixture within the range which satisfies the condition of the above formula (1) with respect to a substance amount of the reactant in the reaction system where the catalyst mixture is used, the effect of the present invention can be more reliably obtained.

Further, from the viewpoint of obtaining the effect of the present invention more reliably, in the catalyst mixture of the present invention described in any one of (N1) to (N3), it is preferable that

(N4) a component of a solvent used in the hydrogenation reaction satisfies the condition of the following equation (3):

$$0.00\% \leq \{100 \text{ x } (Vh/Vs)\} \leq 30.00\% \qquad (3)$$

in the equation (3), Vh being a volume (L) of an introduced water other than the yielded water, and Vs being a volume (L) of the organic solvent.

The present inventors have found that by setting the water content in the reaction system where the catalyst mixture of the present invention is used to be relatively small as mentioned above, the catalyst mixture of the present invention can exhibit the effect of reducing the dehalogenation reaction more reliably. This structure is different from the catalyst described in Patent Document 4 (catalyst having a structure where the Fe oxide is not contained and used in a reaction system where contains water), and shows a remarkable effect obtained through the investigation by the present inventors.

Furthermore, from the same viewpoint, in a case of the catalyst mixture (N4), it is more preferable that

(N5) the water satisfies the condition of the following equation (4):

$$1.00\% \leq \{100 \text{ x } (Vh/Vs)\} \leq 5.00\% \qquad (4)$$

Further, any one of the catalyst mixtures of any one of (N1) to (N5) may further contain

(N6) a dehydrating agent.

When previously mixing the dehydrating agent, the water content can be reduced in the reaction system where the catalyst mixture is used, and there is a case where the effects of the present invention can be obtained easily.

Effect of the invention

The present invention can provide the catalyst mixture which, in a nitro group hydrogenation reaction of an aromatic nitro compound (aromatic halogen nitro compound) having a structure in which nitro groups and halogen atoms are directly bonded as substituents to a ring skeleton of the same ring while separated from each other, is capable of selectively hydrogenating the nitro groups and sufficiently reducing the removal of the halogen atoms from the ring.

Means for Solving the Problem

In the following, preferred embodiments of the present invention are explained in detail.

The catalyst mixture of the present embodiment contains the catalyst and the base. The catalyst contains the support, and the Pt particles and the Fe oxide particles supported on the support. The base has a basicity stronger than at least one aromatic amine obtained as the product from the hydrogenation reaction and having one or more amino groups.

The catalyst is not particularly limited as long as the catalyst contains the support, and the Pt particles and the Fe oxide particles supported on the support.

The support of the catalyst is not particularly limited as long as the support can support the Pt particles and the Fe oxide particles and has a large surface area. Examples include a carbon-based material such as an activated carbon, a pulverized activated carbon, a grassy carbon (GC), a fine carbon, a carbon black, graphite, or a carbon fiber, or a glass-based or a ceramic-based material such as an oxide, and may be properly used.

The specific surface area of the support is preferably 500 $m^3$/g or more, more preferably 800 $m^3$/g or more, and still more preferably 1000 $m^3$/g or more.

In the Fe oxide particles of the catalyst contained in the catalyst mixture, the Fe oxide is not particularly limited, but preferably contains $Fe_2O_3$ as a main component.

The base contained in the catalyst mixture is not particularly limited as long as the base has a basicity stronger than at least one of the aromatic amines having one or more amino groups obtained as the product by hydrogenation reaction, and, in the state of being mixed with the Pt particles and the Fe oxide particles, has stability such that the chemical reaction dese not proceed with these particles at normal temperature and normal pressure. Any of inorganic base and organic base can be used. Further, the base can be properly used in consideration of the combination with the reactant and the solvent in the reaction system to be used. From the viewpoint of availability and the like, preferable examples of the base include sodium carbonate, sodium bicarbonate, potassium carbonate, triethylamine, sodium acetate.

The "aromatic amine having one or more amino groups" desirably is the aromatic halogen amine as the main product, but within the range where the effect of the present invention can be obtained (within the range being allowable in the reaction system to be used), a case where an aromatic amine which is a dehalogenated body is slightly contained may be allowable.

The content of the base contained in the catalyst mixture may be set to an optimum value in the reaction system and reaction conditions where the catalyst mixture is used.

However, as mentioned above, from the viewpoint of obtaining the effect of the present invention more reliably, in the catalyst mixture, it is preferable that the base and a component of a solvent used in the hydrogenation reaction satisfy the condition of the following equation (1):

$$0.90 \leq \{1000 \text{ x } (B/Vs)\} \leq 190.00 \qquad (1)$$

in the equation (1), B being a substance amount (mol) of the base, and Vs being a volume (L) of the organic solvent.

Further, from the viewpoint of obtaining the effect of the present invention more reliably, in the catalyst mixture, it is preferable that the base and the reactant used in the hydrogenation reaction satisfy the condition of the following equation (2):

$$0.35\% \leq \{100 \text{ x } (B/R)\} \leq 75.50\% \qquad (2)$$

in the equation (2), B being a substance amount (mol) of the base, and R being a substance amount (mol) of the reactant.

Further, from the viewpoint of obtaining the effect of the present invention more reliably, in the catalyst mixture, it is preferable that a component of a solvent used in the hydrogenation reaction satisfies the condition of the following equation (3):

$$0.00\% \leq \{100 \text{ x } (Vh/Vs)\} \leq 30.00\% \quad (3)$$

in the equation (3), Vh being a volume (L) of an introduced water other than the yielded water, and Vs being a volume (L) of the organic solvent.

Furthermore, from the same viewpoint, in this case, it is more preferable that the water satisfies the condition of the following equation (4):

$$1.00\% \leq \{100 \text{ x } (Vh/Vs)\} \leq 5.00\% \quad (4)$$

Further, the catalyst mixture may further contain the dehydrating agent.

The dehydrating agent is not particularly limited as long as, in the state of being mixed with the Pt particles and the Fe oxide particles, the agent has stability such that the chemical reaction does not proceed with these substances at normal temperature and normal pressure. From the viewpoint of availability and the like, preferable examples of the dehydrating agent include zeolite, sodium sulfate, magnesium sulfate.

The solvent of the reaction system where the catalyst mixture is used is not particularly limited as long as the solvent has chemical properties capable of dissolving at least a part of the reactant (aromatic halogen nitro compound). Preferred examples include toluene, xylene, benzene, chlorobenzene, dichlorobenzene, and an alcohol having 1 to 3 carbon atoms. A mixture thereof at an optional ratio may be used.

The reactant (aromatic halogen nitro compound) in the reaction system where the catalyst mixture is used is not particularly limited, and it is preferable that the reactant has the structure represented by the following general formula (C1):

[Chemical Formula 1]

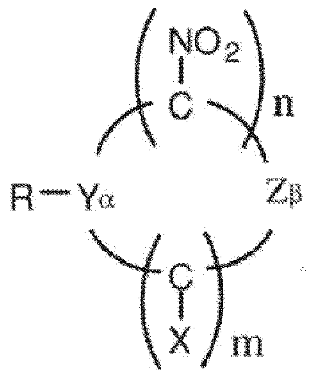

・・・(C1)

In the formula (C1),
n represents an integer of 1 or more,
m represents an integer of 1 or more,
α represents an integer of 0 or more,
β represents an integer of 0 or more, and

$$5 \leq (n + m + \alpha + \beta) \leq 6,$$

R represents hydrogen atom, amino group, hydroxyl group or a mono-valent or more-valent organic group having at least one carbon atom,
X represents any one of halogen atoms,
Y represents, in the case of α = 1, an atom of C, N, O, or S, and, in the case of α ≧ 2, at least two atoms selected from the group consisting of C, N, O and S,
Z represents, in the case of β = 1, an atom of C, N, O, or S, and, in the case of β ≧ 2, at least two atoms selected from the group consisting of C, N, O and S, and
Y and Z may be the same or different from each other.

Here, when $\alpha$ is 2 or more, R may be a divalent organic group bonded to two adjacent Y atoms. In this case, the aromatic nitro compound may have a structure of a condensed ring compound.

In addition, R which is an organic group having mono- or more-valence, may have a structure in which the moiety bonding to $Y\alpha$ is represented by "-O-" or "-S-".

For example, in the formula (C1), in the case of $\alpha = 2$, $Y_1 = C$, $Y_2 = N$, $R = H$, $\beta = 2$, $Z_1 = C$, $Z_2 = C$, $m = 1$, $X = Br$, $n = 1$, $(n + m + \alpha + \beta) = 6$, (C1) is 2-bromo-5-nitropyridine represented by the following formula (C1-1).

[Chemical Formula 2]

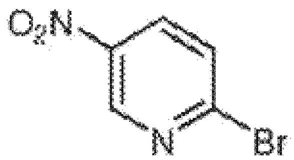

・・・(C1-1)

The reactant (aromatic halogen nitro compound) in the reaction system where the catalyst mixture is used may have the structure represented by the following general formula (C2):

[Chemical Formula 3]

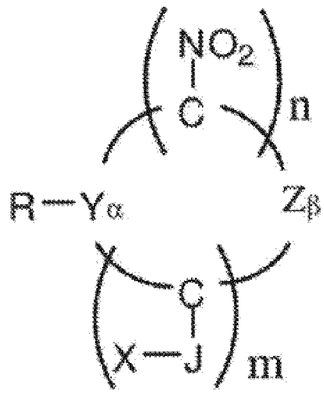

・・・(C2)

In the formula (C2),
n represents an integer of 1 or more,
m represents an integer of 1 or more,
$\alpha$ represents an integer of 0 or more,
$\beta$ represents an integer of 0 or more, and

$$5 \leq (n + m + \alpha + \beta) \leq 6,$$

R represents hydrogen atom, amino group, hydroxyl group or a mono-valent or more-valent organic group having at least one carbon atom,
X represents any one of halogen atoms,
Y represents, in the case of $\alpha = 1$, an atom of C, N, O, or S, and, in the case of $\alpha \geqq 2$, at least two atoms selected from the group consisting of C, N, O and S,
Z represents, in the case of $\beta = 1$, an atom of C, N, O, or S, and, in the case of $\beta \geqq 2$, at least two atoms selected from the group consisting of C, N, O and S,
Y and Z may be the same or different from each other, and
J represents a divalent organic group having one or more carbon atoms, and in the case of $m \geqq 2$, J may be the same or different from each other.

In addition, R which is an organic group having mono- or more-valence, may have a structure in which the moiety bonding to $Y\alpha$ is represented by "-O-" or "-S-".

For example, in the formula (C2), the moiety represented by the "X-J-" may have the following structures

represented by the formulae (C2-1), (C2-2), (C2-3), and (C2-4).

X-C-(C=O)-N- (C2-1)

X-C=C-C- (C2-2)

X-C-C- (C2-3)

[Chemical Formula 4]

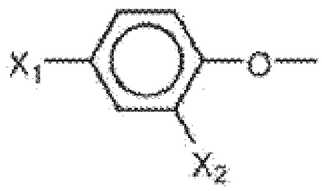

・・・(C2-4)

In the formula (C2-4), $X_1$ and $X_2$ each represent any one of halogen atoms, and $X_1$ and $X_2$ may be the same or different from each other.

<Preparation process of Catalyst Mixture>

The preparation process of the catalyst mixture is not particularly limited, and any combination of known processes can be employed.

As the catalyst contained in the catalyst mixture, for example, a catalyst where the Pt particles and the Fe oxide particles are supported on the support may be obtained by subjecting a dispersion containing a Pt compound, a Fe compound and water to reduction treatment to the support. The supporting of Pt and the supporting of Fe oxide may be carried out at the same time as described above, or one may be carried out first and then the other may be later.

The mixing procedure of the catalyst and the base is also not particularly limited as long as these components can sufficiently mix without causing any undesired chemical reaction.

Further, the mixing procedure of the catalyst, the base and the dehydrating agent is also not particularly limited as long as these components can sufficiently mix without causing any undesired chemical reaction. The order of mixing of the catalyst, the base and the dehydrating agent may be properly set by considering the respective chemical properties or the like.

Furthermore, the catalyst mixture may be solid or liquid. When being solid, it may be powder.

EXAMPLE

In the following, the present invention is explained in detail by referring working examples, but the present invention is not limited by the following working examples.

<Preparation of Catalyst Mixture>

(Example 1)

As the catalyst, a catalyst where the Pt particles and the Fe oxide particles were carried on a carbon support {trade name "NE-01M02", content of Pt: 1.0 wt%, content of Fe: 0.20 wt% available from N. E. CHEMCAT Co. (hereinafter referred to as "Pt-FeOx/C" as necessary)} was prepared.

In the Pt-FeOx/C, the carbon support is an activated carbon (specific surface area based on BET measurement is 900: $m^2/g$), and the Fe oxide particles are $Fe_2O_3$ as a main component ($Fe_2O_3$ is approximately 100% based on XPS analysis).

As the base, a commercially available $Na_2CO_3$ was prepared.

A catalyst mixture was obtained by mixing 127.0 mg (water content: 0.141 mL) of Pt-FeOx/C powder and 1.0 mg of $Na_2CO_3$.

(Example 2) to (Example 5)

The catalyst mixtures of Example 2 to Example 5 were prepared in the same preparation conditions and the same raw materials as Example 1 except that the amount of $Na_2CO_3$ was changed to the value shown in Table 1.

(Example 6)

The catalyst mixture of Example 6 was prepared in the same preparation conditions and the same raw materials as in Example 1 except that 20 mg of a commercially available $K_2CO_3$ was used instead of the amount of $Na_2CO_3$.

(Example 7)

The catalyst mixture of Example 7 was prepared in the same preparation conditions and the same raw materials as in Example 1 except that 72.6 mg of a commercially available $(CH_3CH_2)_3N$ was used instead of the amount of $Na_2CO_3$.

(Example 8) to (Example 14)

The catalyst mixtures of Example 8 to Example 14 were prepared in the same preparation conditions and the same raw materials as Example 3 except that the volume Vh (mL) of water was changed to the value shown in Table 1.

(Comparative Example 1)

As the catalyst of Comparative Example 1, the catalyst consisting of the powder of Pt-FeOx/C which was the same as that in Example 1 was prepared without using a base.

(Comparative Example 2)

The catalyst mixture of Comparative Example 2 was prepared in the same preparation conditions and the same raw materials as in Comparative Example 1 except that 200 mg of a commercially available NaCl was used without using a base.

(Comparative Example 3)

The catalyst mixture of Comparative Example 3 was prepared in the same preparation conditions and the same raw materials as in Comparative Example 2 except that 30 mg of a commercially available $Na_2SO_4$ was used without using a base.

(Comparative Example 4)

Instead of the Pt-FeOx/C used in Example 1, as the catalyst, a catalyst where the Pt particles were carried on a carbon support {trade name "NE-01M00", content of Pt: 1.0 wt%, available from N. E. CHEMCAT Co. (hereinafter referred to as "Pt/C" as necessary)} was prepared.

In the Pt/C, the carbon support is an activated carbon (specific surface area based on BET measurement is 900: $m^2/g$).

As the base, a commercially available $Na_2CO_3$ was prepared.

A catalyst mixture was obtained by mixing 127.0 mg (water content: 0.113 mL) of Pt/C powder and 10.0 mg of $Na_2CO_3$.

(Comparative Example 5)

The catalyst mixture of Comparative Example 2 was prepared in the same preparation conditions and the same raw materials as Comparative Example 1 except that the amount of $Na_2CO_3$ was changed to the value shown in Table 1.

(Comparative Example 6) to (Comparative Example 8)

The catalyst mixtures of Comparative Example 6 to Comparative Example 8 were prepared in the same preparation conditions and the same raw materials as Comparative Example 1 except that the amount of the Pt/C powder to be used was changed to 140.0 mg (water content: 0.123 mL).

(Comparative Example 9)

As the catalyst of Comparative Example 9, the catalyst consisting of the powder of Pt/C which was the same as that in Comparative Example 1 was prepared without using a base.

<Hydrogenation Reaction>

The hydrogenation reaction (hydrogenation reaction of nitro group) represented by the following reaction scheme (C1-11) was achieved by using the catalyst mixtures of Example 1 to Example 14 and Comparative Example 1 to Comparative Example 9.

[Chemical Formula 5]

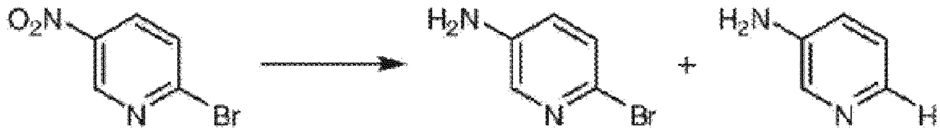

• • • (C1-11)

[Chemical Formula 6]

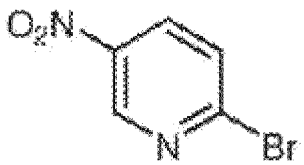

• • • (C1-1)

[Chemical Formula 7]

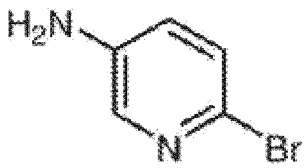

• • • (C1-2)

[Chemical Formula 8]

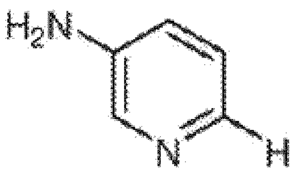

• • • (C1-3)

Here, in the hydrogenation reaction represented by the reaction scheme (C1-11), 2-bromo-5-nitropyridine represented by the formula (C1-1) has a heterocyclic structure having an N atom. Compared to 2-bromo-5-nitrobenzene which has a structure in which the N atom in this heterocyclic ring is replaced with a C atom, 2-bromo-5-nitropyridine is a reactant which promotes not only the main hydrogenation reaction of the nitro group (main product being 5-amino-2-bromopyridine represented by the formula (C1-2)), but also promotes easily the side reaction of the debromination reaction (by-product being 3-aminopyridine represented by the formula (C1-3).

(Reaction Conditions)

Reactant: 2.5 mmol of 2-Bromo-5-nitrobenzene represented by the formula (C1-1)
Solvent: 10 mL of toluene
Pressure of hydrogen: 0.6 MPa
Reaction temperature: 50 °C
Reaction time: 5 hours

In the hydrogenation reaction where the catalyst mixtures of Comparative Example 6 to Comparative Example 8 were used, water was added to each reaction system. The amount of water added to the reaction composition of Comparative Example 6 was adjusted to 0.444 mL, and the amount of water added to the catalyst mixtures of Comparative Example 7 and Comparative Example 8 were adjusted to be 1.000 mL.

The results obtained for the catalyst mixtures of Examples 1 to 14 and Comparative Examples 1 to 9 are shown in Table 1 and Table 2. The formula weight of $Na_2CO_3$ was 106 g ·mol, the formula weight of $K_2CO_3$ was 138.2 g ·mol, and the formula weight of $(CH_3CH_2)_3N$ was 101 g ·mol.

[Table 1]

| | Catalyst structure | Base (other additives) | Base Mass /mg | B Base Substace amount /mmol | Vs Org. Solvent (Toluen) volume/mL | 100x (B/Vs) /mol $L^{-1}$ | R Reactant Substace amount /mmol | 100x (B/R) /mol $L^{-1}$ | Vh Water volume /mL | 100x (Vh/Vs) % |
|---|---|---|---|---|---|---|---|---|---|---|
| Ex.1 | Pt-FeOx/C | $Na_2CO_3$ | 1.0 | 0.0094 | 10.0 | 0.94 | 2.50 | 0.38 | 0.141 | 1.41 |
| Ex.2 | Pt-FeOx/C | $Na_2CO_3$ | 5.0 | 0.0472 | 10.0 | 4.72 | 2.50 | 1.89 | 0.141 | 1.41 |
| Ex.3 | Pt-FeOx/C | $Na_2CO_3$ | 10.0 | 0.0943 | 10.0 | 9.43 | 2.50 | 3.77 | 0.141 | 1.41 |
| Ex.4 | Pt-FeOx/C | $Na_2CO_3$ | 20.0 | 0.1887 | 10.0 | 18.87 | 2.50 | 7.55 | 0.141 | 1.41 |
| Ex.5 | Pt-FeOx/C | $Na_2CO_3$ | 200.0 | 1.8868 | 10.0 | 188.68 | 2.50 | 75.47 | 0.141 | 1.41 |
| Ex.6 | Pt-FeOx/C | $K_2CO_3$ | 20.0 | 0.1447 | 10.0 | 14.47 | 2.50 | 5.79 | 0.141 | 1.41 |
| Ex.7 | Pt-FeOx/C | $(CH_3CH_3)_3N$ | 72.6 | 0.7188 | 10.0 | 71.88 | 2.50 | 23.75 | 0.141 | 1.41 |
| Ex.8 | Pt-FeOx/C | $Na_2CO_3$ | 10.0 | 0.0943 | 10.0 | 9.43 | 2.50 | 3.77 | 0.460 | 4.60 |
| Ex.9 | Pt-FeOx/C | $Na_2CO_3$ | 10.0 | 0.0943 | 10.0 | 9.43 | 2.50 | 3.77 | 1.136 | 11.36 |
| Ex.10 | Pt-FeOx/C | $Na_2CO_3$ | 10.0 | 0.0943 | 10.0 | 9.43 | 2.50 | 3.77 | 2.000 | 20.00 |
| Ex.11 | Pt-FeOx/C | $Na_2CO_3$ | 10.0 | 0.0943 | 10.0 | 9.43 | 2.50 | 3.77 | 2.200 | 22.00 |
| Ex.12 | Pt-FeOx/C | $Na_2CO_3$ | 10.0 | 0.0943 | 10.0 | 9.43 | 2.50 | 3.77 | 2.800 | 28.00 |
| Ex.13 | Pt-FeOx/C | $Na_2CO_3$ | 10.0 | 0.1048 | 9.0 | 10.48 | 2.50 | 3.77 | 1.620 | 18.00 |
| Ex.14 | Pt-FeOx/C | $Na_2CO_3$ | 10.0 | 0.1048 | 9.0 | 10.48 | 2.50 | 3.77 | 1.800 | 20.00 |

(continued)

| | Catalyst structure | Base (other additives) | Base Mass /mg | B Base Substace amount /mmol | Vs Org. Solvent (Toluen) volume/mL | 100x (B/Vs) /mol $L^{-1}$ | R Reactant Substace amount /mmol | 100x (B/R) /mol $L^{-1}$ | Vh Water volume /mL | 100x (Vh/Vs) % |
|---|---|---|---|---|---|---|---|---|---|---|
| Com.Ex. 1 | Pt-FeOx/C | Non | 0.0 | 0.0000 | 10.0 | 0.00 | 2.50 | 0.00 | 0.141 | 1.41 |
| Com.Ex. 2 | Pt-FeOx/C | Non (NaCl) | 0.0 | 0.0000 | 10.0 | 0.00 | 2.50 | 0.00 | 0.141 | 1.41 |
| Com.Ex. 3 | Pt-FeOx/C | Non ($Na_2SO_4$) | 0.0 | 0.0000 | 10.0 | 0.00 | 2.50 | 0.00 | 0.141 | 1.41 |
| Com.Ex. 4 | Pt/C | $Na_2CO_3$ | 10.0 | 0.0943 | 10.0 | 9.43 | 2.50 | 3.77 | 0.113 | 1.13 |
| Com.Ex. 5 | Pt/C | $Na_2CO_3$ | 5.0 | 0.0472 | 10.0 | 4.72 | 2.50 | 1.89 | 0.113 | 1.13 |
| Com.Ex. 6 | Pt/C | $Na_2CO_3$ | 10.0 | 0.0943 | 10.0 | 9.43 | 2.50 | 3.77 | 0.567 | 5.67 |
| Com.Ex. 7 | Pt/C | $Na_2CO_3$ | 10.0 | 0.0943 | 10.0 | 9.43 | 2.50 | 3.77 | 1.123 | 11.23 |
| Com.Ex. 8 | Pt/C | $Na_2CO_3$ | 10.0 | 0.0943 | 10.0 | 9.43 | 2.50 | 3.77 | 1.123 | 11.23 |
| Com.Ex. 9 | Pt/C | Non | 0.0 | 0.0000 | 10.0 | 0.00 | 2.50 | 0.00 | 0.113 | 1.13 |

[Table 2]

| | Conversion % | Selectivity % 5-amino-2-bromopyridine main product | Selectivity % 3-aminopyridine by-product (DeBr body) |
|---|---|---|---|
| Ex.1 | 100 | 90.8 | 9.2 |
| Ex.2 | 100 | 98.1 | 1.9 |
| Ex.3 | 100 | 99.5 | 0.5 |
| Ex.4 | 100 | 99.7 | 0.3 |
| Ex.5 | 100 | 99.6 | 0.4 |
| Ex.6 | 100 | 99.6 | 0.4 |
| Ex.7 | 100 | 98.2 | 1.8 |
| Ex.8 | 100 | 98.4 | 1.6 |
| Ex.9 | 100 | 96.0 | 4.0 |
| Ex.10 | 100 | 92.8 | 7.2 |
| Ex.11 | 100 | 92.9 | 7.1 |
| Ex.12 | 100 | 91.3 | 8.7 |
| Ex.13 | 100 | 94.0 | 6.0 |
| Ex.14 | 100 | 92.8 | 7.2 |
| Com.Ex.1 | 100 | 81.5 | 18.5 |
| Com.Ex.2 | 100 | 79.5 | 20.5 |
| Com.Ex.3 | 100 | 84.5 | 15.5 |
| Com.Ex.4 | 100 | 84.7 | 15.3 |
| Com.Ex.5 | 100 | 72.4 | 27.6 |
| Com.Ex.6 | 100 | 82.9 | 17.1 |
| Com.Ex.7 | 100 | 66.5 | 33.5 |
| Com.Ex.8 | 100 | 42.1 | 57.9 |
| Com.Ex.9 | 100 | 48.9 | 51.1 |

From the results shown in Table 2, when the reaction system using the catalyst mixtures of Example 1 to Example 14 which satisfies the constitution of the present invention was compared with the reaction system using the catalyst mixtures of Comparative Example 1 to Comparative Example 9, it has been clear that the debromination reaction to 3-aminopyridine represented by the formula (C1-3) was sufficiently reduced, and the selectivity of the desired 5-amino-2-bromopyridine represented by the formula (C1-2) was improved.

In particular, when the reaction system using the catalyst mixtures of Example 1 to Example 14 which satisfies the constitution of the present invention was compared with the reaction system using the catalyst mixture of Comparative Example 1 which has the structure similar to that of Patent Document 3 described as a prior art document (reaction system which has a catalyst containing a Pt component and an iron oxide component, and does not contain a base), it has been clear that the debromination reaction to 3-aminopyridine represented by the formula (C1-3) was sufficiently reduced.

Further, when the reaction system using the catalyst mixtures of Example 1 to Example 14 which satisfies the constitution of the present invention was compared with the reaction system using the catalyst mixture of Comparative Example 6 which has the structure similar to that of Patent Document 4 described as a prior art document (reaction system which has a catalyst containing a Pt component, and a base and water is added (in Example 1 of Patent Document 4, $100 \times (Vh / Vs) = 100 \times$ (amount of added water 30 mL + water contained in the catalyst 1.5 mL) / toluene 675 mL = 4.6%), it has been clear that the debromination reaction to 3-aminopyridine represented by the formula (C1-3) was sufficiently reduced.

From the aforementioned results, it has been clear that, in a nitro group hydrogenation reaction of an aromatic

nitro compound (aromatic halogen nitro compound) having a structure in which nitro groups and halogen atoms are directly bonded as substituents to a ring skeleton of the same ring while separated from each other, the catalyst mixture of the present examples is capable of selectively hydrogenating the nitro groups and sufficiently reducing the removal of the halogen atoms from the ring.

Industrial Applicability

The catalyst mixture of the present invention has the catalyst activities that, in a nitro group hydrogenation reaction of an aromatic nitro compound (aromatic halogen nitro compound) having a structure in which nitro groups and halogen atoms are directly bonded as substituents to a ring skeleton of the same ring while separated from each other, is capable of selectively hydrogenating the nitro groups and sufficiently reducing the removal of the halogen atoms from the ring.

Therefore, the present invention contributes to the development of efficient mass production technology of the aromatic halogen amine which is an important raw material of medicines, dyes, insecticides and herbicides, and further contributes to the development of the industries of pharmaceuticals, dyes, insecticides, herbicides.

## Claims

1. A catalyst mixture comprising a catalyst which is used in a hydrogenation reaction of at least one among one or more nitro groups present in a reactant, which is an aromatic nitro compound having a structure in which one or more nitro groups and one or more halogen atoms are directly bonded as substituents to a ring skeleton of the same ring while separated from each other, which comprises:

   a catalyst and a base, and
   the catalyst comprises a support, and Pt particles and Fe oxide particles supported on the support, and
   the base has a basicity stronger than at least one aromatic amine obtained as a product from the hydrogenation reaction and having one or more amino groups.

2. The catalyst mixture according to claim 1, wherein the base and a component of a solvent used in the hydrogenation reaction satisfy the condition of the following equation (1):

$$0.90 \leq \{1000 \text{ x } (B/Vs)\} \leq 190.00 \qquad (1)$$

   in the equation (1), B being a substance amount (mol) of the base, and Vs being a volume (L) of the organic solvent.

3. The catalyst mixture according to claim 1 or 2, wherein the base and the reactant used in the hydrogenation reaction satisfy the condition of the following equation (2):

$$0.35\% \leq \{100 \text{ x } (B/R)\} \leq 75.50\% \qquad (2)$$

   in the equation (2), B being a substance amount (mol) of the base, and R being a substance amount (mol) of the reactant.

4. The catalyst mixture according to any one of claims 1 to 3, wherein a component of a solvent used in the hydrogenation reaction satisfies the condition of the following equation (3):

$$0.00\% \leq \{100 \text{ x } (Vh/Vs)\} \leq 30.00\% \qquad (3)$$

   in the equation (3), Vh being a volume (L) of an introduced water other than the yielded water, and Vs being a volume (L) of the organic solvent.

5. The catalyst mixture according to claim 4, wherein the water satisfies the condition of the following equation (4):

$$1.00\% \leq \{100 \times (Vh/Vs)\} \leq 5.00\% \quad (4)$$

**6.** The catalyst mixture according to any one of claims 1 to 5, further comprising a dehydrating agent.

## INTERNATIONAL SEARCH REPORT

International application No.
PCT/JP2017/005582

A. CLASSIFICATION OF SUBJECT MATTER
*B01J23/89*(2006.01)i, *C07C209/36*(2006.01)i, *C07C209/38*(2006.01)i

According to International Patent Classification (IPC) or to both national classification and IPC

B. FIELDS SEARCHED

Minimum documentation searched (classification system followed by classification symbols)
B01J23/89, C07C209/36, C07C209/38

Documentation searched other than minimum documentation to the extent that such documents are included in the fields searched

| | | | |
|---|---|---|---|
| Jitsuyo Shinan Koho | 1922-1996 | Jitsuyo Shinan Toroku Koho | 1996-2017 |
| Kokai Jitsuyo Shinan Koho | 1971-2017 | Toroku Jitsuyo Shinan Koho | 1994-2017 |

Electronic data base consulted during the international search (name of data base and, where practicable, search terms used)
JSTPlus(JDreamIII), JST7580(JDreamIII), JSTChina(JDreamIII)

C. DOCUMENTS CONSIDERED TO BE RELEVANT

| Category* | Citation of document, with indication, where appropriate, of the relevant passages | Relevant to claim No. |
|---|---|---|
| X<br>Y | JP 03-005442 A (E.I. Du Pont de Nemours & Co.), 11 January 1991 (11.01.1991), claims 6 to 10; examples 1 to 7 & US 4990663 A1 examples 1 to 7; claims & EP 398542 A2 | 1,3<br>2,4-6 |
| Y | JP 48-086830 A (Mitsui Toatsu Chemicals, Inc.), 15 November 1973 (15.11.1973), claims; page 4, upper right column, lines 15 to 16; example 8; table 1 & US 4070401 A1 column 4, lines 18 to 19; example 8; table 1; claims | 2,4-6 |

☒ Further documents are listed in the continuation of Box C. ☐ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 08 March 2017 (08.03.17) | 21 March 2017 (21.03.17) |

| Name and mailing address of the ISA/ | Authorized officer |
|---|---|
| Japan Patent Office<br>3-4-3,Kasumigaseki,Chiyoda-ku,<br>Tokyo 100-8915,Japan | Telephone No. |

Form PCT/ISA/210 (second sheet) (January 2015)

**INTERNATIONAL SEARCH REPORT**

International application No.
PCT/JP2017/005582

C (Continuation). DOCUMENTS CONSIDERED TO BE RELEVANT

| Category* | Citation of document, with indication, where appropriate, of the relevant passages | Relevant to claim No. |
|---|---|---|
| Y | JP 2000-302738 A (Wakayama Seika Kogyo Co., Ltd.), 31 October 2000 (31.10.2000), claims 1 to 2; paragraph [0015] (Family: none) | 6 |
| A | JP 55-015690 A (E.I. Du Pont de Nemours & Co.), 02 February 1980 (02.02.1980), entire text & US 4212824 A1 entire text | 1-6 |
| A | JP 2008-516882 A (Peking University), 22 May 2008 (22.05.2008), entire text & US 2009/0124834 A1 entire text & WO 2006/042453 A1 & EP 1826180 A1 | 1-6 |
| A | JP 02-045452 A (Hoechst AG.), 15 February 1990 (15.02.1990), entire text & US 5041671 A1 entire text & EP 347796 A2 | 1-6 |
| A | JP 10-076161 A (Degussa AG.), 24 March 1998 (24.03.1998), entire text & US 6316381 B1 entire text & US 2001/0056035 A1 & EP 820808 A1 | 1-6 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- JP H5271105 B
- JP H671178 B
- US 4212824 A
- JP H245452 B


### Non-patent literature cited in the description


- **SODA ; CHLORIDE.** *General Remarks Detoxification of Aromatic chloride compound,* January 2005
- **Y. UKISU et al.** *Appl. Catal. A: General.,* 2004, vol. 271, 165-170
- **Y. UKISU et al.** *Appl. Catal. B: Environ.,* 2000, vol. 27 (97), 97-104
- **R. H. MIZZONI et al.** *J. Am, Chem. Soc.,* 1951, vol. 73, 1873
- **M. M. ROBIMSON.** *J. Am, Chem. Soc.,* 1958, vol. 80, 6254
- **E. V. BROWN.** *J. Org. Chem.,* 1965, vol. 30, 1607
- Revised 5th Edition Chemical Handbook Basic II. The Chemical Society of Japan
- **L. D. PETTIT ; K. J. POWELL.** Stability Constants Database. Academic Software, 1997